# EUROPEAN PATENT SPECIFICATION

(11) **EP 1 796 698 B1**
(45) Date of publication and mention of the grant of the patent: **02.11.2011**
(21) Application number: 05788495.9
(22) Date of filing: 21.09.2005
(51) Int. Cl.: A61K 35/74, C12Q 1/02, A23C 9/123, A23L 1/30

(54) **STRAIN OF LACTOBACILLUS ACIDOPHILUS HAVING ANALGESIC PROPERTIES IN THE GASTROINTESTINAL SYSTEM**
STAMM VON LACTOBACILLUS ACIDOPHILUS MIT ANALGETISCHEN EIGENSCHAFTEN IM GASTROINTESTINALSYSTEM
SOUCHE DE LACTOBACILLUS ACIDOPHILUS A PROPRIETES ANALGESIQUES DANS LE SYSTEME GASTRO-INTESTINAL

(30) Priority: 21.09.2004 FR 0409966
(43) Date of publication of application: 20.06.2007
(73) Proprietor: DANISCO A/S, 1001 Copenhagen K. (DK)
(72) Inventor: DESREUMAUX, Pierre, F-59800 Lille (FR); CARCANO, Didier, F-75005 Paris (FR)
(74) Representative: Touati, Catherine
(86) International application number: PCT/EP2005/010880
(87) International publication number: WO 2006/032542

(56) References cited:
- WO-A-03/013558
- WO-A-2004/052462
- US-B1- 6 203 797
- HALPERN G M ET AL: "Treatment of irritable bowel syndrome with Lacteol Fort: a randomized, double-blind, cross-over trial." THE AMERICAN JOURNAL OF GASTROENTEROLOGY. AUG 1996, vol. 91, no. 8, August 1996 (1996-08), pages 1579-1585, XP009046865 ISSN: 0002-9270
- MATAR C GOULET J: "beta-Casomorphin 4 from milk fermented by a mutant of lactobacillus helveticus" INTERNATIONAL DAIRY JOURNAL, ELSEVIER APPLIED SCIENCE, BARKING,, GB, vol. 6, 1996, pages 383-397, XP002960565 ISSN: 0958-6946
- ROKKA T ET AL: "RELEASE OF BIOACTIVE PEPTIDES BY ENZYMATIC PROTEOLYSIS OF LACTO-BACILLUS GG FERMENTED UHT MILK" MILCHWISSENSCHAFT, VV GMBH VOLKSWIRTSCHAFTLICHER VERLAG. MUNCHEN, DE, vol. 52, no. 12, 1997, pages 675-677, XP000733203 ISSN: 0026-3788
- KOSTYRA, ELZBIETA ET AL: "Procedure for the evaluation of the opioid activity of milk products" POLISH JOURNAL OF FOOD AND NUTRITION SCIENCES , 12(SI 1), 39-42 CODEN: PJFSE7; ISSN: 1230-0322, 2003, XP009046853

## Description

Among microorganisms, in particular among bacteria, some have a positive influence on the immune system, in particular the lactic bacteria and bifidobacteria, and are described as "probiotic" bacteria or strains.

Generally, by probiotic bacterium or strain is meant a non-pathogenic microorganism which, ingested live, exercises a beneficial effect on the host's health or physiology. These probiotic strains generally have the ability to survive the passage through the upper part of the digestive tract. They are non-pathogenic, non-toxic and exercise their beneficial effect on health on the one hand via ecological interactions with the resident flora in the digestive tract, and on the other hand via their ability to influence the immune system in positive manner via the "GALT" (gut-associated lymphoid tissue). Depending on the definition of probiotics, these bacteria, when given in a sufficient number, have the ability to progress live through the intestine, however they do not cross the intestinal barrier and their primary effects are therefore felt in the lumen and/or the wall of the gastrointestinal tract. They then form part of the resident flora during the administration period. This colonization (or temporary colonization) allows the probiotic bacteria to exercise a beneficial effect, such as the repression of potentially pathogenic micro-organisms present in the flora and interactions with the immune system of the intestine.

The probiotic strains most used, in particular in dairy products, are principally bacteria and yeasts of the following genera: *Lactobacillus spp., Streptococcus spp., Enterococcus spp., Bifidobacterium spp. and Sacharomyces spp..* Among the probiotic effects recorded for these bacteria, there can be mentioned for example the improvement of lactose tolerance, prevention or treatment of gastrointestinal and urogenital infections, reduction of cancers, reduction of the blood cholesterol level. However, it must be stressed that not all the strains of the genera described above, taken individually, have these effects, but only some of them, which must be carefully selected.

In order to meet the requirements of industrialists, it has become necessary to find strains or mixtures of strains which are effective and make it possible to provide a solution to pain experienced at gastrointestinal level, and often expressed as intestinal discomfort, in particular irritable bowel syndrome (IBS), or pain caused by inflammation at gastrointestinal level, in particular during colitis or diarrhoea.

Document WO 03/013558 discloses a method of treating pain and discomfort of actinic colitis resulting from radiation therapy, said method comprising administering to a person in need of same said method comprising administering to a person in need of same an effective amount of a probiotic dietary or pharmaceutical composition comprising protective streptococci, lactobacilli and bifidobacteria selected from the group consisting of *Streptococcus thermophilus, Lactobacillus casei, Lactobacillus plantarum, Lactobacillus acidophilus, Lactobacillus delbruecki, Bifidobacterium longum, Bifidobacterium infantis and Bifidobacterium bifidum.* in a daily amount of from at least 100x10⁹ to 3,600x10⁹ bacteria cells.

Thus the problem which the present invention proposes to resolve is to provide a strain of probiotic bacteria having analgesic properties.

To this end the present invention proposes the use of at least one strain of *Lactobacillus acidophilus* to prepare a support administered to humans or animals for an analgesic purpose in the gastrointestinal system.

The present invention is defined by the claims.

The invention also proposes an in vitro process for selecting a microorganism to prepare a support administered to humans or animals for an analgesic purpose in the gastrointestinal system comprising the following stages:
i) bringing the microorganism to be tested into contact with at least one epithelial cell;
ii) detecting the expression of the opioid receptors and/or cannabinoid receptors in least one epithelial cell;

The invention has the advantage of proposing virtues which are indisputable and which will expand the range of available strains.

The invention has another advantage, of being able to be used for a therapeutic or prophylactic purpose in the gastrointestinal system.

The invention is particularly advantageous when it is administered to humans or animals for a therapeutic or prophylactic purpose in the gastrointestinal system, in particular in the reduction of pain experienced in the case of irritable bowel syndrome or in the case of pain caused by inflammatory reactions or in the regulation of intestinal inflammation.

The invention is also advantageous as it makes it possible to regulate the intestinal transit by modulating secretion and digestive motricity, when it is administered to humans or animals for a therapeutic or prophylactic purpose.

The present invention also has the advantage of preserving all its properties when it is incorporated into a pharmaceutically acceptable support or into a food product.

Other advantages and characteristics of the invention will appear more clearly on reading the following description and examples given purely as an illustration and non-limitatively.

A subject of the present invention is the use of at least one strain of *Lactobacillus acidophilus* to prepare a support administered to humans or animals for an analgesic purpose in the gastrointestinal system.

The *Lactobacillus acidophilus* used according to the invention is a gram-positive strain. Advantageously it is a catalase-negative strain, with a homofermentative metabolism giving rise to the production of lactic acid.

The *Lactobacillus acidophilus* used according to the invention can also produce a bacteriocin, such as for example lactacin, active against other microorganisms.

Preferably, it is a *Lactobacillus acidophilus* having a good resistance to pepsin, under acid pH conditions, a good resistance to pancreatin and a good tolerance to the bile salts.

Preferably, a *Lactobacillus acidophilus* described as "hydrophobic" will be used, i.e. one having a strong affinity to polar or non-polar hydrophobic organic solvents, such as for example n-decane, chloroform, hexadecane or xylene.

The *Lactobacillus acidophilus* strains preferred according to the invention are *Lactobacillus acidophilus* PTA-4797 and *Lactobacillus acidophilus* NCFM. The *Lactobacillus acidophilus* PTA-4797 strain of *Lactobacillus acidophilus* has been registered by Rhodia Chimie, 26, quai Alphonse Le Gallo, 92 512 BOULOGNE-BILLANCOURT Cedex France, in accordance with the Budapest Treaty at the American Type Culture Collection (ATCC), where it is recorded under registration number PTA-4797. This strain is disclosed in WO2004052462.

Within the framework of the present use according to the invention, analgesia in the gastrointestinal system is advantageously mediated via the opioid receptors and/or cannabinoid receptors.

The opioid receptors described are three in number: δ, κ, and µ. They belong to the superfamily of receptors coupled to the G proteins which are composed of seven transmembrane helices. The intracellular part of the receptor is in contact with the G protein which is associated with it and which can vary depending on the type of agonists used, but predominantly the proteins Gαi₃> Gαi₂>Gαi₁.

The opioid receptors, in particular the µ receptor, have several functions. The main one is an analgesic role demonstrated by the use of β-endorphin- or morphine-type agonists specific to this receptor passing the haematomeningeal barrier. The second function of this receptor in the digestive tract is to reduce the intestinal transit by inhibiting secretion and digestive motricity. Finally, the opioid receptors are also involved in the regulation of intestinal inflammation.
The µ receptor for the opioids is present in the central nervous system but also at the periphery. Its presence has been detected in the majority of the vital organs of the human body: the spleen, liver, kidneys, small intestine and colon in particular in the intestinal nervous system in the neurons of the submucous and mesenteric plexus, but also, *in vitro,* in the lymphocytes, monocytes/macrophages and epithelial cells.

The cannabinoid receptors, called CB1 and CB2, belong to the superfamily of receptors coupled to the G proteins which are composed of seven transmembrane helices. They are expressed essentially by the central and peripheral nervous system for CB1 and the immune response cells for CB2. In humans, there are two endogenous ligands of these cannabinoid receptors, which are naturally produced by the intestinal epithelial cells.

The cannabinoid receptors CB1 expressed by the enteric nervous system would be the cause of a slowing-down of the peristalsis of the stomach and small intestine and an inhibition of gastric secretion. Other anti-diarrhoetic and anticancer functions of the cannabinoid receptors are presumed.

Within the framework of the present use according to the invention, analgesia in the gastrointestinal system is preferably mediated via the µ receptors for the opioids and/or the CB1 receptors and/or the CB2 receptors.

The support employed during the use according to the invention is preferably a pharmaceutically acceptable support or a food product.

By pharmaceutically acceptable support is meant *inter alia* a support in the form of compressed tablets, tablets, capsules, ointments, suppositories or drinkable solutions.

Preferably, the support employed during the use according to the invention is a food product such as a food supplement, a drink or a powder based on milk. Preferably it is a dairy product of animal or vegetable origin.

By dairy product is meant a medium comprising milk of animal and/or vegetable origin. As milk of animal origin there can be mentioned cow's, sheep's, goat's or buffalo's milk. As milk of vegetable origin there can be mentioned any fermentable substance of vegetable origin which can be used according to the invention, in particular originating from soybeans, rice or cereals.

Still more preferably the support employed according to the invention is a fermented milk or humanized milk.

The strain of *Lactobacillus acidophilus* used to prepare a support according to the invention can be in the form of a bacterial suspension, before or after freezing, in the form of concentrates, either in dry, lyophilized or frozen form. Whatever the form used, the strain can be frozen.

The strain of *Lactobacillus acidophilus* used to prepare a support according to the invention can contain different additives added during its drying or during its lyophilization.

The strain of *Lactobacillus acidophilus* used according to the invention can comprise from 10⁶ to 10¹² CFU of bacteria/g of support, and more particularly from 10⁸ to 10¹² CFU of bacteria/g of support. CFU stands for "colony-forming units". By gram of support is meant preferably the food product or the pharmaceutically acceptable support, and preferably 10⁹ to 10¹² CFU/g for the lyophilized form.

A subject of the invention is also an in vitro process for selecting a microorganism to prepare a support administered to humans or animals for an analgesic purpose in the gastrointestinal system comprising the following stages:
i) bringing the microorganism to be tested into contact with at least one epithelial cell;
ii) detecting the expression of the opioid receptors and/or cannabinoid receptors in least one epithelial cell;
The epithelial cell or cells used during stages i) or ii) preferably come from the cell line ATCC HTB-38, commonly called HT-29 line or from the cell line Caco-2. These are cancer colon cell lines. They can also be isolated and purified cells from biopsies of items from operations on humans.

Stage i) is carried out preferably using from 10⁸ to 10¹² CFU of microorganisms to be tested with at least one epithelial cell.

The contact period, during stage i), can vary from 0 hour to 24 hours, and is preferably 3 hours.

Generally, the bringing into contact with the cells according to stage i) is carried out under standard temperature, modified-atmospheres and sterility conditions well known to a person skilled in the art, in particular under *in vitro* epithelial cell culture conditions.

Stage ii) of the selection process according to the invention is carried out by preferably detecting the expression of the µ receptors for the opioids (MOR) and/or the CB1 receptors and/or the CB2 receptors. Typically, the expression of only one receptor can be detected: MOR alone, the CB1 receptor alone or the CB2 receptor alone. Alternatively, the expression of two receptors can be detected: MOR and CB1 receptor; MOR and CB2 receptor; CB1 receptor and CB2 receptor. Alternatively the expression of three receptors can be detected: MOR, CB1 receptor and CB2 receptor.

Stage ii) of the selection process according to the invention is carried out by preferably detecting the expression, and optionally its level, of the messenger RNA of the opioid receptors and/or cannabinoid receptors, for example by PCR *inter alia* by quantitative PCR or by immunohistochemistry. Other techniques well known to a person skilled in the art for the detection of mRNA and its measurement can be used.
Figure 1 represents, as a function of time, the messenger RNA expression kinetics of the µ receptors for the opioids, expressed in the ATCC HTB-38 epithelial cells during stimulation by 4 different microorganisms.
Figure 2 represents, as a function of time, the messenger RNA expression kinetics of the CB1 receptors, expressed in the ATCC HTB-38 epithelial cells during stimulation by 3 different microorganisms.
Figure 3 represents, as a function of time, the messenger RNA expression kinetics of the CB2 receptors, expressed in the ATCC HTB-38 epithelial cells during stimulation by 3 different microorganisms.
Figure 4 represents the MOR mRNA expression under different conditions.
Figure 5 represents the colonic TNF alpha mRNA expression in untreated mice and in *L. acidophilus* treated mice.
Figure 6 represents the colonic KC mRNA expression in untreated mice and in *L. acidophilus* treated mice.
Figure 7 represents the colonic IL-1 beta mRNA expression in untreated mice and in *L*. *acidophilus* treated mice.
Figures 8, 10, 12 represent immunostained epithelial cells.
Figures 9, 11, 13 represent the percentage of immunostained epithelial cells.

The following examples illustrate the invention without limiting its scope.

### EXAMPLES:

### Example 1

### 1/ Preparation of the epithelial cells:

The colon cancer cell line HT-29 (ATCC HTB-38) is cultured in DMEM medium with respectively 20 and 10% foetal calf serum at 37°C in an atmosphere containing 5% CO₂. The ATCC HTB-38 cell line is brought into contact with the different strains of microorganisms to be tested for 1; 3; 4; 8; 18 or 24 hours. The ATCC HTB-38 epithelial cells are then recovered and immersed in liquid nitrogen in order to make it possible to quantify the mRNA and the protein of the µ receptors for the opioids and/or cannabinoid receptors.

### 2/ Detection and quantification of the messenger RNAs of the µ receptors for the opioids andlor cannabinoid receptors:

### Real-time PCR:

The total RNA of the epithelial cell lines in culture is isolated by use of a column extraction kit (Macherey-Nagel). In brief, the cells are ground in lysis buffer containing 1% β-mercapto-ethanol then passed onto a first type of column allowing the elimination of all the waste. After treatment with DNAse, the RNA will be retro-transcribed into complementary. DNA amplified by PCR in real time (ABPrism 7000, Perkin) at a hybridization temperature of 60°C using primers specific to the µ receptors for the opioids or cannabinoid receptors:
- for the µ receptors for the opioids (MOR) (Sense: ATgCCAgTgCTCATCATTAC, Anti-sense: gATCCTTCgAAgATTCCTgTCCT) and for the reference gene: β-actin (S:TCACCCACACTgTgCCCATCTACgA, AS: CAgCggAACCgCTCATTgCCAATg);
- for the CB1 cannabinoid receptors, Sense CCT AGA TGG CCT TGC AGA TAC C; CB1 Anti-sense TGT CAT TTG AGC CCA CGT ACA G; CB2 Sense GCT AAG TGC CCT GGA GAA CGT; CB2 Anti-sense TCA GCC CCA GCC AAG CT.

### 3/ Results:

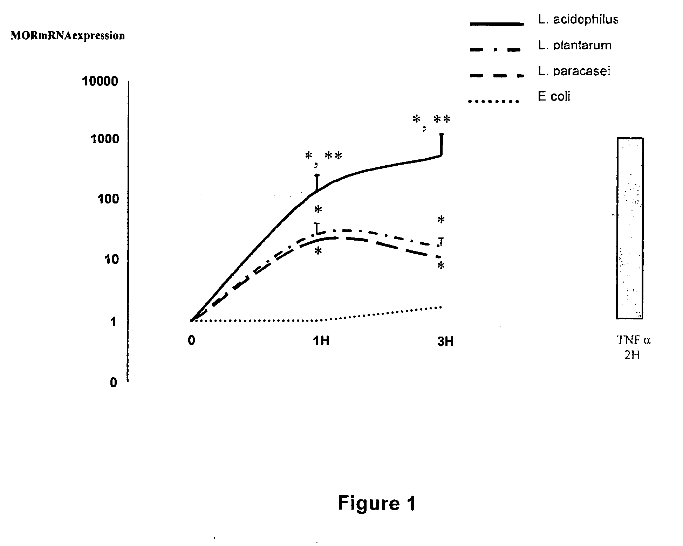

The results are presented in Figures 1 to 3. The results are expressed by the ratio between the target gene (β-actin) / µ receptors for the opioids (MOR) and/or cannabinoid receptors (CB1, CB2).

The µ receptors for the opioids and/or cannabinoid receptors are expressed in the ATCC HTB-38 epithelial cells line (Figures 1 to 3).

Some microorganisms such as the strain *Lactobacillus* are capable of inducing the expression of the *mRNA* of the µ receptors for the opioids and/or cannabinoid receptors.

The results show a significant induction of the µ receptors for the opioids and/or cannabinoid receptors by the epithelial cells in culture.

This induction is particularly strong with the strain *Lactobacillus acidophilus.*

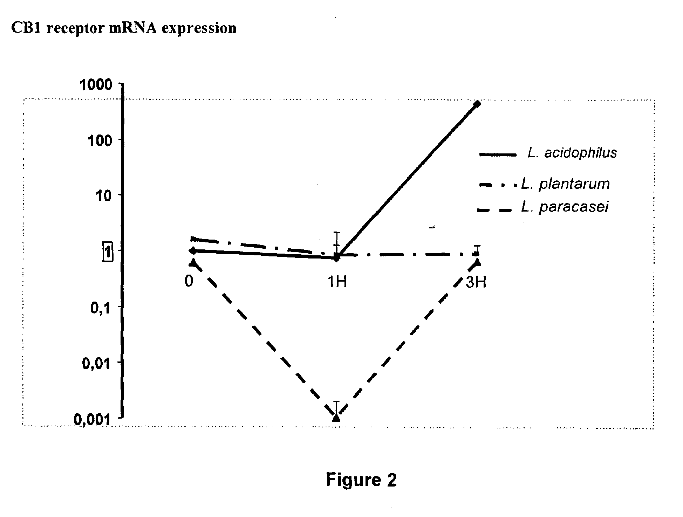

Figures 1 and 2 show that after 3 hours' incubation of the epithelial cells with *Lactobacillus acidophilus* an approximately 1000-fold increase is observed in the basal expression level of the mRNA of the µ receptors for the opioids (Figure 1) and CB1 (Figure 2).

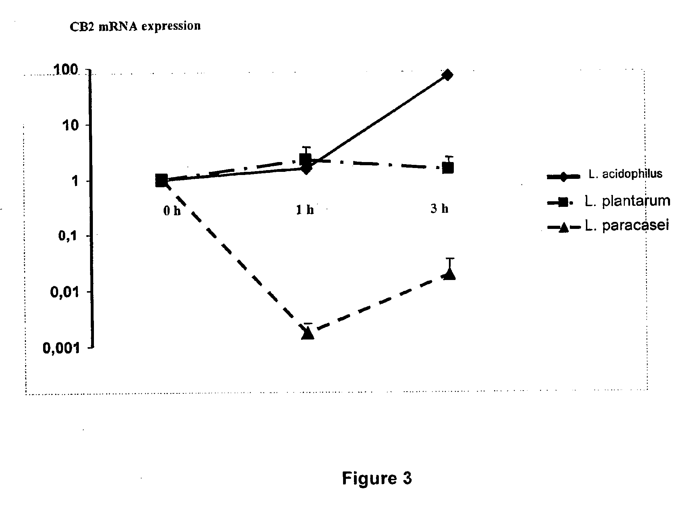

Figure 3 shows that after 3 hours' incubation of the epithelial cells with *Lactobacillus acidophilus* an approximately 100-fold increase is observed in the basal expression level of the mRNA of the CB2 receptors (Figure 3).

Conversely, no induction of the µ receptors for the opioids was detected with a commensal *E. coli* strain (Figure 1). The induction of the opioid receptors and/or cannabinoid receptors by the *Lactobacillus acidophilus* strain is of the same order of magnitude as that obtained by TNF-α at a dose of 10 ng/ml.

### Example 2

### 1/Materials & methods

**Bacterial strain**. *Lactobacillus acidophilus* NCFM strain according to the present invention was grown anaerobically in deMan, Rogosa, Sharpe (MRS) broth (Becton Dickinson) overnight at 37°C. For in vitro experiments, bacteria were used when they reached the exponential phase. Cultures were assessed for purity by Gram staining prior to animal inoculation.

**Animals and experimental infection**. Animal experiments were performed in accredited establishments at Institut Pasteur from Lille according to governmental guidelines. Balb/c mice were housed five per cage and had free access to standard mouse chow and tap water under a 12-h daylight cycle. Animals received 10⁹ CFU of *Lactobacillus acidophilus* strain, which were resuspended in 0.5% CMC (CarboxyMethyl Cellulose, Sigma), once a day by gastric gavage during fourteen days. Animals were killed by cervical dislocation. All colons were excised from animals and cut into two parts. One part was fixed overnight in 4% paraformaldehyde acid and embedded in paraffin. The second part of the colon was used for quantification of mu-opioid receptor (MOR), cannabinoid receptors (CB1 and CB2), and inflammatory cytokines TNFα, KC, and IL-1β mRNA.

**Induction of TNBS colitis and study design**. Since the expression of MOR is regulated by inflammation (Philippe D et al, JCI 2003; Pol O et al, Mol Pharmacol 2001; Pol O et al, Curr Top Med Chem 2004), we used TNBS-induced colitis as a positive control. Animal experiments were performed in accredited establishments at Institut Pasteur from Lille according to governmental guidelines. Animals were housed five per cage and had free access to standard mouse chow and tap water. For colitis induction, mice were anesthetized for 90-120 minutes and received an intrarectal administration of TNBS (40 µl, 150 mg/kg) dissolved in a 1:1 mixture of 0.9% NaCl with 100% ethanol. Control mice received a 1:1 mixture of 0.9% NaCl with 100% ethanol or a saline solution using the same technique. Animals were sacrificed 4 days after TNBS administration.

### Quantitative real-time PCR

Total RNA was isolated from whole colonic tissues using Rneasy kit (Macherey Nagel, Hoerdt, France) according to the manufacturer's instructions. RNA quantification was performed using spectrophotometry. After treatment at 37°C for 30 min with 20-50 units of RNase-free DNase I (Roche Diagnostics Corporation, Indianapolis, IN, USA), oligo-dT primers (Roche Diagnostics Corporation, Indianapolis, USA) were used to synthesize single-stranded cDNA. mRNAs were quantified using SYBR green Master Mix (Applera, Courtaboeuf, France) with specific mouse oligonucleotides (see table I), in a GeneAmp Abiprism 7000 (Applera, Courtaboeuf, France). In each assay, calibrated and no-template controls were included. Each sample was run in triplicate. SYBR green dye intensity was analyzed using the Abiprism 7000 SDS software (Applera, Courtaboeuf, France). All results were normalized to the unaffected housekeeping gene β-actin.

**Table I:**

| **Genes** | Primer sequences (5' → 3') |
|---|---|
| β-actin | S: 5'-gggTCAgAAggATTCCTATg-3' |
| | AS : 5' ggTCTCAAACATgATCTggg-3' |
| MOR | S: 5' - CCG GCA GCC CTT CCA-3' |
| | AS: 5'- GAG GCC CAC TAC ACA CAC GAT -3' |
| CB1 | S: 5' .GCC CGC ATG GAC ATT AGG-3' |
| | AS: 5'AGG GCC CCA GCA GAT GA -3' |
| CB2 | S: 5' - CTC AAT TTT TCT GGT CCC TAT G-3' |
| | AS: 5-AGT CTG GCA CCG CTA AAC AAG-3' |
| TNF alpha | S:5'-TgggAgTAgACAAggTACAACCC-3' |
| | AS : 5' CATCTTTCTCAAAATTCgAgTgACAA-3' |
| IL-1 beta | S:5'-gATCCACACTCTCCAgCTgCA -3' |
| | AS : 5'-CAACCAACAAgTgATATTCTCCATg-3' |
| KC | S:5'-ggCgCCTATCgCCAATg -3' |
| | AS : 5'-CTggATgTTCTTgAggTgAATCC -3' |

### MOR-CB1-CB2 immunohistochemistry

Immunohistochemistry was performed on colon embedded-paraffin sections of mice receiving the *Lactobacillus acidophilus* strain. Untreated animals were used as controls. After permeabilisation during 5 min in PBS containing 0.1 % triton X-100 at 4°C, sections were incubated for 15 min with 1.5 % goat normal serum and 15 min with blocking buffer (1% BSA in milk) to minimize nonspecific adsorption of the antibodies. The tissues were subsequently incubated with the rabbit polyclonal primary antibody directed against CB1 (1:200, Cayman Chemical, Ann Arbor, USA) or CB2 (1:10, Alpha Diagnostic, San Antonio, USA) or MOR (1:500, Diasorin, Antony, France) for 2 to 12 hours at room temperature. Sections were then incubated for 1 h at room temperature with Alexa 488 goat anti-rabbit IgG conjugated to FITC fluorochrome (dilution 1:100, Dako Laboratories, Trappes, France). Between each stage, sections were rinsed twice for 5 min in PBS containing 0.05% triton X-100. Then slides were counterstained with Hoescht solution (0.125 mg/mL) and mounted for microscopy. Negative controls consisted of staining with normal rabbit serum instead of specific antibody. Immunofluorescence was revealed under a fluorescence microscope (Leica, Bensheim, Germany). The number of MOR, CB1 and CB2 immunoreactive epithelial cells was counted in five different high power fields (HPF) and expressed per 100 epithelial cells.

### 2/Results:

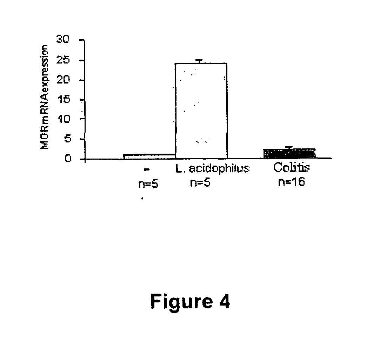

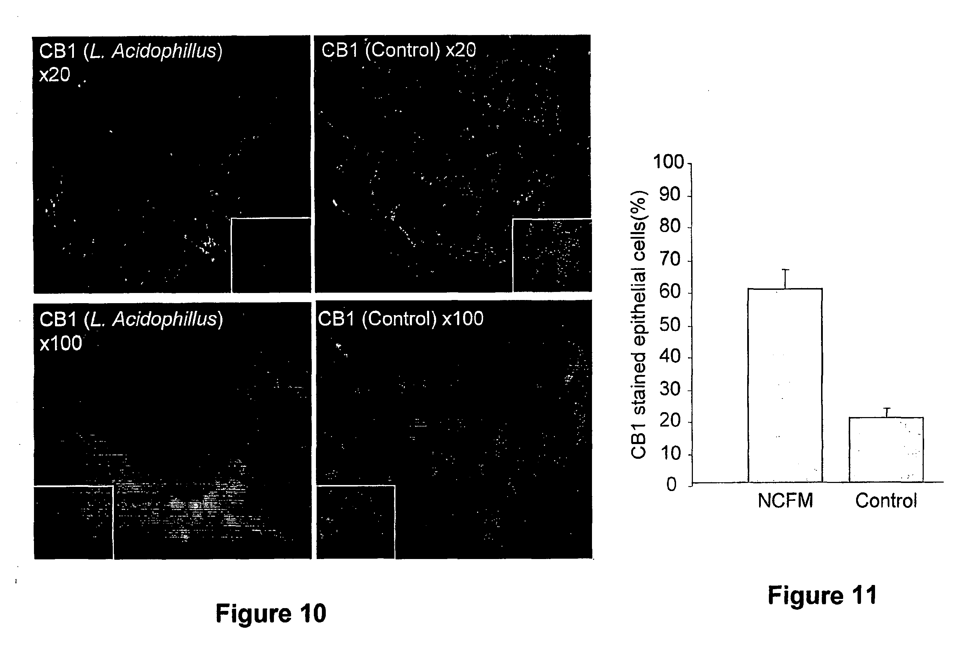

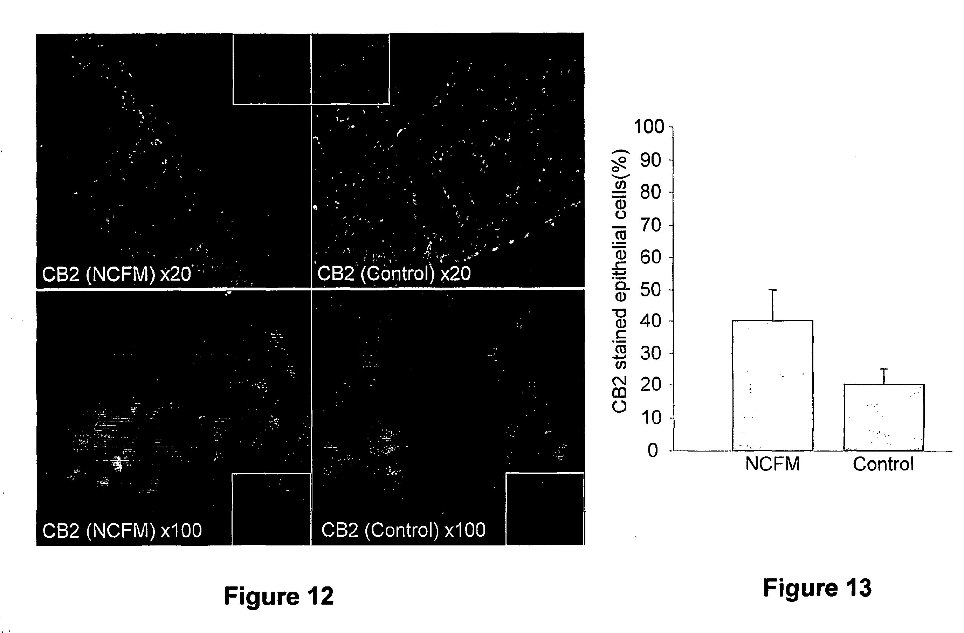

The results are presented in Figures 4 to 13.

### 1) Lactobacillus acidophilus induces in vivo MOR mRNA expression in the colon of mice (Figure 4).

After two weeks of *Lactobacillus acidophilus* administration (10⁹ CFU per day), a 24 fold increase of MOR mRNA expression was found in the colon by comparison with untreated animals (p<0.05). This induction of MOR mRNA by the *Lactobacillus acidophilus* strain was more important compared to the two fold induction found in our positive controls corresponding to colitis induced by TNBS (Figure 4).

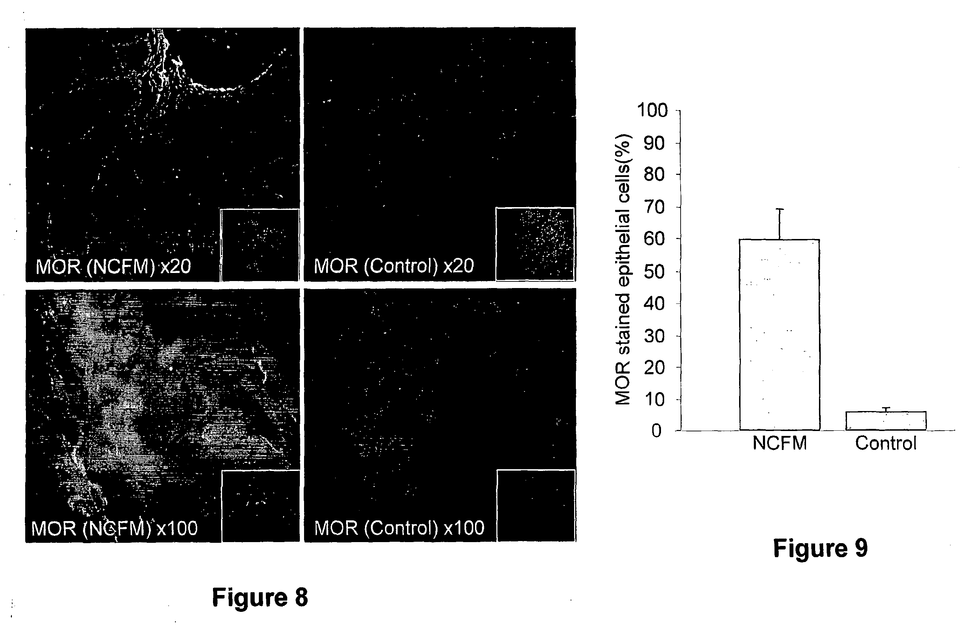

### 2) Lactobacillus acidophilus induce in vivo expression of MOR, CB1 and CB2 in colonic epithelial cells of mice (Figures 8-13)

To evaluate at the translational level the induction of MOR, CB1 and CB2 specifically on epithelial cells of the colon, we performed immunohistochemistry using antibodies directed specifically against these receptors. In all sections, epithelial stained cells for MOR (60±10 vs 5±3%), CB1 (60±8 vs 20±4%) or CB2 (40±7 vs 20±5%) were significantly more numerous in mice receiving the *Lactobacillus acidophilus* strain compared to control mice (Figures 8-13). The green staining was mainly localized in epithelial cells located at the surface of the epithelium, in contact with luminal bacteria (Figures 8-13). Controls omitting the first antibody or the use of an irrelevant antibody were negative.

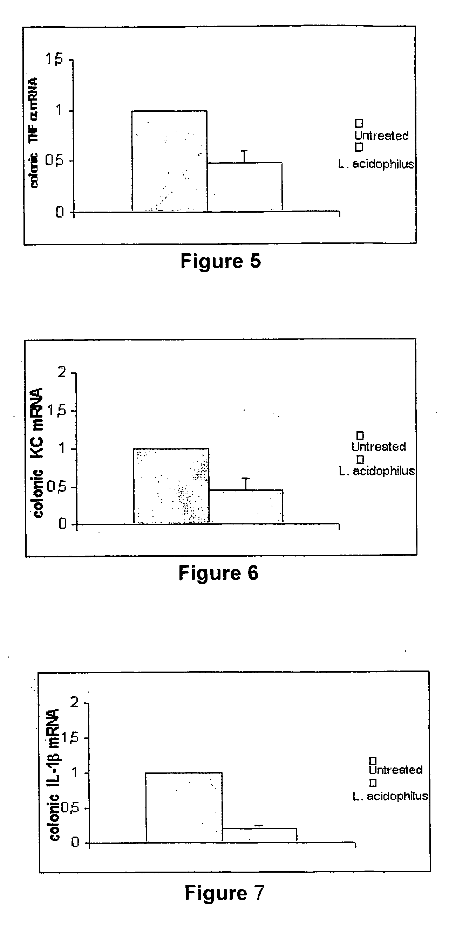

### 3) Lactobacillus acidophilus administration is associated with a decrease inflammatory cytokine expression in the colon of mice (Figures 5-7)

In order to evaluate if the *Lactobacillus acidophilus*-induced expression of MOR, CB1 and CB2 by colonic epithelial cells may have functional significance in mice, we compared the mRNA levels of different inflammatory cytokines in untreated mice and animals receiving the *Lactobacillus acidophilus* strain during 14 days. More than 50% decreased expression of inflammatory cytokines TNFα, KC and IL-1β mRNA levels was observed in *Lactobacillus acidophilus*-treated animals compared to controls suggesting that *Lactobacillus acidophilus* strain reduces the physiological expression of inflammatory cytokines in the colon of mice through at least in part an overexpression of MOR, CB1 and CB2 by epithelial cells.

### Example 3

### 1/Materials and Methods

*In vitro,* epithelial colonic cells HT-29 or Caco-2 were incubated during 0 to 6 hours with different probiotics or bacteria (100 bacteria/cell): *L*. *acidophilus* (NCFM), *L*. *salivarius* (UCC118), *L. paracasei* (LPC37), commensal *E*. *coli,* adherent-invasive *E*. *coli* (LF82). The role of the NFkappaB pathway in the induction of the expression of the mu-opioid receptor (MOR) and of the cannabinoid receptors (CB1 and CB2) by probiotics was tested by pre-treating the HT-29 cells by a specific inhibitor, the caffeic acid phenetyl ester (CAPE). TNFalpha (10 ng/ml, 2hours), which is an inductor of the expression of G coupled proteins, was used as a positive control. After the selection of the most efficient probiotic for inducing *in vitro* the expression of the receptors (MOR, CB1 and CB2 receptors), an *in vivo* study was made with Balb/c mice (n=10) by oral administration of 10⁹ CFU of probiotic during 15 days. The expression of MOR, CB1 and CB2 was assessed in the epithelial cells and in the colon of mice by real time PCR and by immunohistochemistry. The inflammatory cytokines (TNF alpha, KC and IL-1 beta) mRNA was assessed by real time PCR in the colon of the mice.

### 2/ Results

Only the strains *L*. *acidophilus* and *L*. *salivarius,* were able to quickly induce, already from the first hour of incubation, a strong MOR expression in the epithelial cells. This expression induction was similar to the expression induction observed with TNF alpha induced cells. For CB1 (848±180 vs 229±55, p<0.05) and CB2 (1498±333 vs 341±163, p<0.01), only *L*. *Acidophilus* induced the expression of these receptors. The inhibition of the NFkappaB pathway by CAPE did not induce modifications of the expression of MOR by epithelial cells stimulated with *L*. *acidophilus. In vivo,* the administration of *L*. *acidophilus* strongly induced the expression of MOR mRNA (24±0.75, p<0.01) at the colonic level. The study by immunohistochemistry confirmed *in vivo* the induction of the MOR, CB1 and CB2 expression by colonic cells in animals receiving *L*. *acidophilus.* The induction of these receptors is linked with a diminution of at least 50% of the expression of colonic inflammatory cytokines.

### 3/ Conclusion

*L. acidophilus* is the most efficient strain for inducing the expression of MOR, CB1 and CB2 *in vitro* in epithelial cell lines and *in vivo* in the colon.

## Claims

1. *Lactobacillus acidophilus* for use in the analgesic treatment of the gastrointestinal system of humans or animals, wherein said *Lactobacillus acidophilus* is not in the form of a mixture with lactic bacteria.

2. *Lactobacillus acidophilus* for use in the analgesic treatment of the gastrointestinal system of humans or animals, wherein said *Lactobacillus acidophilus* is the sole active ingredient.

3. *Lactobacillus acidophilus* for use in the analgesic treatment of the gastrointestinal system of humans or animals according to claim 1, or 2 wherein said *Lactobacillus acidophilus* induces the expression of the µ opioid receptor (MOR) and cannabinoid receptors CB1 and CB2 in colonic epithelial cells.

4. *Lactobacillus acidophilus* for use in the analgesic treatment of the gastrointestinal system of humans or animals, wherein said *Lactobacillus acidophilus* is the strain registered at the ATCC under the number PTA-4797 and also known as *Lactobacillus acidophilus* NCFM.

5. In vitro Process for selecting a microorganism to prepare a support administered to humans or animals for an analgesic purpose in the gastrointestinal system comprising the following stages:
i) bringing the microorganism to be tested into contact with at least one epithelial cell;
ii) detecting the expression of the opioid receptors and/or cannabinoid receptors in at least one epithelial cell;
wherein the microorganism to be tested is selected if it induces the expression of the µ opioid receptor (MOR) and cannabinoid receptors CB1 and CB2.

6. Process according to claim 5 **characterized in that** at least one epithelial cell of stage i) or ii) comes from the cell line ATCC HTB-38 or from the cell line Caco-2.

7. Process according to one of claims 5 to 6 **characterized in that** stage ii) is carried out by detecting the µ receptors for the opioid and the CB1 receptors and the CB2 receptors.

8. Process according to one of claims 5 to 7 **characterized in that** stage ii) is carried out by detecting the expression of the messenger RNA of the opioid receptors and cannabinoid receptors CB 1 and CB2.

## Patentansprüche

1. *Lactobacillus acidophilus* zur Verwendung in der schmerzstillenden Behandlung des Magen-Darm-Systems von Menschen oder Tieren, wobei besagter *Lactobacillus acidophilus* nicht in Form einer Mischung mit Milchsäurebakterien vorliegt.

2. *Lactobacillus acidophilus* zur Verwendung in der schmerzstillenden Behandlung des Magen-Darm-Systems von Menschen oder Tieren, wobei besagter *Lactobacillus acidophilus* der einzig aktive Wirkstoff ist.

3. *Lactobacillus acidophilus* zur Verwendung in der schmerzstillenden Behandlung des Magen-Darm-Systems von Menschen oder Tieren gemäß Anspruch 1 oder 2, wobei besagter *Lactobacillus acidophilus* die Expression des µ Opioidrezeptors (MOR) und der Cannabinoidrezeptoren CB1 und CB2 in den Darmepithelzellen induziert.

4. *Lactobacillus acidophilus* zur Verwendung in der schmerzstillenden Behandlung des Magen-Darm-Systems von Menschen oder Tieren, wobei besagter *Lactobacillus acidophilus* der Stamm ist, der bei der ATCC unter der Nummer PTA-4797 registriert ist und auch als *Lactobacillus acidophilus* NCFM bekannt ist.

5. In-vitro-Verfahren zur Selektion eines Mikroorganismus zur Herstellung eines Trägers, der Menschen oder Tieren für schmerzstillende Zwecke im Magen-Darm-System verabreicht wird, umfassend die folgenden Stufen:
i) Inkontaktbringen des zu testenden Mikroorganismus mit mindestens einer Epithelzelle;
ii) Detektieren der Expression der Opioidrezeptoren und/oder Cannabinoidrezeptoren in mindestens einer Epithelzelle;
wobei der zu testende Mikroorganismus danach ausgewählt ist, ob dieser die Expression des µ Opioidrezeptors (MOR) und der Cannabinoidrezeptoren CB1 und CB 2 induziert.

6. Verfahren gemäß Anspruch 5, **dadurch gekennzeichnet, dass** mindestens eine Epithelzelle der Stufen i) oder ii) aus der Zelllinie ATCC HTB-38 oder der Zelllinie Caco-2 stammt.

7. Verfahren gemäß einem der Ansprüche 5 bis 6, **dadurch gekennzeichnet, dass** die Stufe ii) mittels Detektion der µ Rezeptoren für das Opioid und der CB1 Rezeptoren und die CB2 Rezeptoren durchgeführt wird.

8. Verfahren gemäß einem der Ansprüche 5 bis 7, **dadurch gekennzeichnet, dass** die Stufe ii) mittels Detektion der Expression der Messenger RNA der Opioidrezeptoren und Cannabinoidrezeptoren CB1 und CB2 ausgeführt wird.

## Revendications

1. *Lactobacillus acidophilus* pour son utilisation dans le traitement analgésique du système gastro-intestinal d'humains ou d'animaux, dans lequel ledit *Lactobacillus acidophilus* n'est pas sous la forme d'un mélange avec des bactéries lactiques.

2. *Lactobacillus acidophilus* pour son utilisation dans le traitement analgésique du système gastro-intestinal d'humains ou d'animaux, dans lequel ledit *Lactobacillus acidophilus* est le seul ingrédient actif.

3. *Lactobacillus acidophilus* pour son utilisation dans le traitement analgésique du système gastro-intestinal d'humains ou d'animaux selon la revendication 1 ou 2, dans lequel ledit *Lactobacillus acidophilus* induit l'expression du récepteur opioïde µ (MOR) et des récepteurs cannabinoïdes CB1 et CB2 dans les cellules épithéliales du côlon.

4. *Lactobacillus acidophilus* pour son utilisation dans le traitement analgésique du système gastro-intestinal d'humains ou d'animaux, dans lequel ledit *Lactobacillus acidophilus* est la souche déposée à l'ATCC sous le numéro PTA-4797 et est également connue sous le nom de *Lactobacillus acidophilus* NCFM.

5. Procédé *in vitro* de sélection d'un micro-organisme pour préparer un support administré à des humains ou des animaux à des fins analgésiques dans le système gastro-intestinal, comprenant les étapes suivantes :
i) la mise en contact du micro-organisme à tester avec au moins une cellule épithéliale ;
ii) la détection de l'expression des récepteurs opioïdes et/ou des récepteurs cannabinoïdes dans au moins une cellule épithéliale ;
dans lequel le micro-organisme à tester est choisi s'il induit l'expression du récepteur opioïde µ (MOR) et des récepteurs cannabinoïdes CB1 et CB2.

6. Procédé selon la revendication 5, **caractérisé en ce qu'**au moins une cellule épithéliale de l'étape i) ou ii) provient de la lignée cellulaire ATCC HTB-38 ou de la lignée cellulaire Caco-2.

7. Procédé selon l'une quelconque des revendications 5 à 6, **caractérisé en ce que** l'étape ii) est effectuée en détectant les récepteurs µ pour l'opioïde et les récepteurs CB1 et les récepteurs CB2.

8. Procédé selon l'une quelconque des revendications 5 à 7, **caractérisé en ce que** l'étape ii) est effectuée en détectant l'expression de l'ARN messager des récepteurs opioïdes et des récepteurs cannabinoïdes CB1 et CB2.


### SEQUENCE LISTING

<110> DANISCO A/S
<120> strain of Lactobacillus acidophilus having analgesic properties in the gastrointestinal system
<130> BCT050283
   <150> FR0409966
<151> 2004-09-21
<160> 22
<170> PatentIn version 3.3
<210> 1
   <211> 20
   <212> DNA
   <213> artificial
<220>
   <223> oligonucleotide as primer
<400> 1
   atgccagtgc tcatcattac 20
<210> 2
   <211> 23
   <212> DNA
   <213> artificial
<220>
   <223> oligonucleotide as primer
<400> 2
   gatccttcga agattcctgt cct 23
<210> 3
   <211> 25
   <212> DNA
   <213> artificial
<220>
   <223> oligonucleotide as primer
<400> 3
   tcacccacac tgtgcccatc tacga 25
<210> 4
   <211> 24
   <212> DNA
   <213> artificial
<220>
   <223> oligonucleotide as primer
<400> 4
   cagcggaacc gctcattgcc aatg 24
<210> 5
   <211> 22 -
   <212> DNA
   <213> artificial
<220>
   <223> oligonucleotide as primer
<400> 5
   cctagatggc cttgcagata cc 22
<210> 6
   <211> 22
   <212> DNA
   <213> artificial
<220>
   <223> oligonucleotide as primer
<400> 6
   tgtcatttga gcccacgtac ag 22
<210> 7
   <211> 21
   <212> DNA
   <213> artificial
<220>
   <223> oligonucleotide as primer
<400> 7
   gctaagtgcc ctggagaacg t 21
<210> 8
   <211> 17
   <212> DNA
   <213> artificial
<220>
   <223> oligonucleotide as primer
<400> 8
   tcagccccag ccaagct 17
<210> 9
   <211> 20
   <212> DNA
   <213> artificial
<220>
   <223> oligonucleotide as primer
<400> 9
   gggtcagaag gattcctatg 20
<210> 10
   <211> 20
   <212> DNA
   <213> artificial
<220>
   <223> oligonucleotide as primer
<400> 10
   ggtctcaaac atgatctggg 20
<210> 11
   <211> 15
   <212> DNA
   <213> artificial
<220>
   <223> oligonucleotide as primer
<400> 11
   ccggcagccc ttcca 15
<210> 12
   <211> 21
   <212> DNA
   <213> artificial
<220>
   <223> oligonucleotide as primer
<400> 12
   gaggcccact acacacacga t 21
<210> 13
   <211> 18
   <212> DNA
   <213> artificial
<220>
   <223> oligonucleotide as primer
<400> 13
   gcccgcatgg acattagg 18
<210> 14
   <211> 17
   <212> DNA
   <213> artificial
<220>
   <223> oligonucleotide as primer
<400> 14
   agggccccag cagatga 17
<210> 15
   <211> 22
   <212> DNA
   <213> artificial
<220>
   <223> oligonucleotide as primer
<400> 15
   ctcaattttt ctggtcccta tg 22
<210> 16
   <211> 21
   <212> DNA
   <213> artificial
<220>
   <223> oligonucleotide as primer
<400> 16
   agtctggcac cgctaaacaa g 21
<210> 17
   <211> 23
   <212> DNA
   <213> artificial
<220>
   <223> oligonucleotide as primer
<400> 17
   tgggagtaga caaggtacaa ccc 23
<210> 18
   <211> 26
   <212> DNA
   <213> artificial
<220>
   <223> oligonucleotide as primer
<400> 18
   catctttctc aaaattcgag tgacaa 26
<210> 19
   <211> 21
   <212> DNA
   <213> artificial
<220>
   <223> oligonucleotide as primer
<400> 19
   gatccacact ctccagctgc a 21
<210> 20
   <211> 25
   <212> DNA
   <213> artificial
<220>
   <223> oligonucleotide as primer
<400> 20
   caaccaacaa gtgatattct ccatg 25
<210> 21
   <211> 17
   <212> DNA
   <213> artificial
<220>
   <223> oligonucleotide as primer
<400> 21
   ggcgcctatc gccaatg 17
<210> 22
   <211> 23
   <212> DNA
   <213> artificial
<220>
   <223> oligonucleotide as primer
<400> 22
   ctggatgttc ttgaggtgaa tcc 23